# EUROPEAN PATENT SPECIFICATION

(11) **EP 2 740 511 B1**
(45) Date of publication and mention of the grant of the patent: **04.07.2018**
(21) Application number: 13194446.4
(22) Date of filing: 26.11.2013
(51) Int. Cl.: A61M 25/01, A61B 17/12, A61M 25/00, A61M 25/06, A61M 5/00

(54) **Microcatheter**
Mikrokatheter
Micro-cathéter

(30) Priority: 07.12.2012 US 201213707655
(43) Date of publication of application: 11.06.2014
(73) Proprietor: Covidien LP, Mansfield, MA 02048 (US)
(72) Inventor: Eskuri, Alan, Irvine, CA 92620 (US)
(74) Representative: Gray, James

(56) References cited:
- WO-A1-99/20326
- WO-A2-00/13734
- WO-A2-2008/011166
- US-A- 5 782 797
- US-A- 5 882 334
- US-A1- 2010 030 102
- US-B1- 6 622 367

## Description

### BACKGROUND

### 1. Technical Field

The present disclosure generally relates to microcatheters, and, in particular, a dual-lumen microcatheter.

### 2. Description of Related Art

Stroke is a common cause of death and disability. Hemorrhagic stroke accounts for 20% of the annual stroke population. Hemorrhagic stroke often occurs due to rupture of an aneurysm or arteriovenous malformation (AVM), causing bleeding into the brain tissue and resultant infarction of brain tissue. The remaining 80% of strokes are due to ischemia that occurs due to occlusion of a blood vessel that deprives brain tissue of oxygen-carrying blood. Ischemic strokes are often caused by emboli or pieces of thrombotic tissue that have dislodged and traveled from other body sites, or from the cerebral vessels themselves, to occlude in the narrow cerebral arteries more distally.

Intravascular treatments for stroke are well known. Aneurysms or AVMs may be treated with liquid embolic compositions, embolic coils, and flow diversion devices. Similarly, ischemic stroke is intravascularly treated with thrombectomy devices. To reach the aneurysm or occlusion microcatheter sand microguidewires must be employed, but often the column support of these microcatheters is not strong enough to navigate through the distal reaches of the neurovasculature to effectively treat these sites. Often guide catheters are employed to act as a conduit to help support microcatheter access. In addition, to visualize the relative position of these devices and progress of the treatment, contrast medium is often used. Guide catheters, positioned adjacent a proximal portion of the microcatheter, are typically used to deliver the contrast medium. The proximal location of the guide catheter relative the microcatheter, however, necessitates the delivery of a large volume of the contrast medium to enable sufficient contrast medium to reach the treatment site. In thrombectomy procedures, the proximal guide catheter is used to aspirate the vessel during material extraction. WO99/20326 discloses a multiple lumen catheter. US5782797 discloses a therapeutic infusion device. WOOO/13734 discloses a method for re-perfusion of oxygenated blood. US6622367 discloses an intravascular device. WO2008/011166 discloses a device and method for removing obstructions from a cerebral vessel. US5882334 discloses a balloon delivery catheter assembly with adjustable balloon positioning. US2010/030102 discloses an active delivery and flow redirection device.

It would be useful to have a microcatheter with increased stability and a second lumen to allow for localized contrast delivery or aspiration.

It would be useful to have a microcatheter with increased proximal support and stability combined with distal catheter flexibility which can be varied depending on the patient and procedure and has a second lumen to allow for localized delivery.

### SUMMARY

The present disclosure is directed to a, as described in claim 1, comprising a first flexible tubular body and a second flexible tubular body. The first flexible tubular body defines a longitudinal axis, has a proximal end, a distal end and a first lumen extending at least partially therethrough. The second flexible tubular body extends substantially parallel to the longitudinal axis along at least a portion of its length and has a second lumen extending at least partially therethrough. The first lumen and the second lumen are coaxially disposed along at least a majority of the length of the second lumen. A distal end of the first lumen extends farther distally than a distal end of the second lumen by a distance x. The first flexible tubular body and the second flexible tubular body are threadably connected, such that the first and second flexible tubular bodies may be rotated relative to each other to alter the distance x. The second flexible tubular body defines a distal exit port in fluid communication with the second lumen, the second flexible tubular body being configured to deliver a fluid via the distal exit port when the first lumen and the second lumen are coaxially disposed.

In disclosed embodiments, the distance x is between about 2 cm and about 10 cm. (e.g., between about 5 cm and about 10 cm).

In disclosed embodiments, the microcatheter also includes a contrast medium disposed in fluid communication with the second lumen and the second lumen is configured for delivery of the contrast medium.

In disclosed embodiments, the distal end of the second flexible tubular body is tapered.

In disclosed embodiments, an outer diameter of the second flexible tubular body is between about 0.060 inches (1.524mm) and about 0.120 inches (3.048mm).

In disclosed embodiments, the microcatheter further comprises a tip body detachably connected to the first tubular body via a coupling.

In disclosed embodiments, the distal end of the second flexible tubular body includes a plurality of exit ports.

In disclosed embodiments, the distal end of the second flexible tubular body includes an annular exit port.

The present disclosure also describes a microcatheter comprising a first flexible tubular body and a second flexible tubular body. Here, the first flexible tubular body defines a longitudinal axis, has a proximal end, a distal end and a first lumen extending at least partially therethrough. The second flexible tubular body extends substantially parallel to the longitudinal axis along at least a portion of its length and has a second lumen extending at least partially therethrough. A distal end of the second lumen is spaced a longitudinal distance x from a distal end of the first lumen, and the distance x is adjustable.

In a disclosed example, the distance x is adjustable from about 2 cm to about 10 cm.

In a disclosed example, the microcatheter also includes a contrast medium disposed in fluid communication with the second lumen and the second lumen is configured for delivery of the contrast medium.

In a disclosed example, the distance x is adjustable via a mechanical structure disposed adjacent a proximal portion of the microcatheter.

The present disclosure also describes a microcatheter comprising a first flexible tubular body, a second flexible tubular body. The first flexible tubular body defines a longitudinal axis, has a proximal end, a distal end and a first lumen extending at least partially therethrough. The second flexible tubular body extends substantially parallel to the longitudinal axis along at least a portion of its length and has a second lumen extending at least partially therethrough. The first lumen and the second lumen are coaxially disposed along at least a majority of the length of the second flexible tubular body. The contrast medium is disposed in fluid communication with the second lumen, and the second lumen is configured for delivery of the contrast medium.

In disclosed embodiments, the second lumen is defined within a sheath. Here, the first flexible tubular body and the sheath are made of the same material. Here, the first flexible tubular body and the sheath have different durometers. In other disclosed embodiments, the first flexible tubular body and the sheath are made of different materials.

The present disclosure also describes a microcatheter comprising a first flexible tubular body and a second flexible tubular body. Here, the first flexible tubular body defines a longitudinal axis, has a proximal end, a distal end and a first lumen extending at least partially therethrough. The second flexible tubular body extends substantially parallel to the longitudinal axis along at least a portion of its length and has a second lumen extending at least partially therethrough. A distal end of the second lumen is spaced a longitudinal distance x from a distal end of the first lumen, and the distance x is between about 2 cm and about 10 cm.

The present disclosure also describes a method of accessing a vascular site. The method comprises providing a microcatheter comprising a first flexible tubular body and a second flexible tubular body. The first flexible tubular body defines a longitudinal axis, has a proximal end, a distal end and a first lumen extending at least partially therethrough. The second flexible tubular body extends substantially parallel to the longitudinal axis along at least a portion of its length and has a second lumen extending at least partially therethrough. A distal end of the second flexible tubular body is spaced a longitudinal distance x from a distal end of the first flexible tubular body, and the
distance x is adjustable. The method also comprises positioning a portion of the microcatheter within a patient, inserting a guidewire through the first lumen, and injecting a contrast medium through the second lumen.

In disclosed examples of the method, the distance x is adjustable from about 2 cm to about 10 cm.

In disclosed examples of the method, the first lumen and the second lumen are coaxially disposed along at least a majority of an entire length of the second lumen.

### BRIEF DESCRIPTION OF THE DRAWINGS

Embodiments of the present disclosure will be readily appreciated by reference to the drawings wherein:
Figure 1 is a side view of a microcatheter in accordance with embodiments of the present disclosure;
Figure 2 is a perspective view of the microcatheter of Figure 1;
Figure 2A is a perspective view of an alternate embodiment of the microcatheter of Figure 2;
Figure 3 is a longitudinal cross-sectional view of the microcatheter along line 3-3 of Figure 2, and illustrates a guidewire extending therethrough;
Figure 4 is a schematic view of the microcatheter in use adjacent an aneurysm within the vasculature of a patient illustrating coils being ejected from a first lumen of the microcatheter and contrast medium that was ejected from a second lumen of the microcatheter;
Figure 4A is a schematic view of the microcatheter in use adjacent an aneurysm within the vasculature of a patient illustrating a flow diversion device deployed from a first lumen of the microcatheter and contrast medium that was ejected from a second lumen of the microcatheter; and
Figure 5 is a schematic view of the microcatheter in use adjacent an occlusion within the vasculature of a patient illustrating a stentreiver device deployed from a first a lumen of the microcatheter and a second lumen of the microcatheter being used for aspiration.

### DESCRIPTION

In the following description, the terms "proximal" and "distal" as used herein refer to the relative position of the microcatheter in a lumen. The "proximal" or "trailing" end of the microcatheter is the microcatheter segment extending outside the body closest to the clinician. The "distal" or "leading" end of the microcatheter is the microcatheter segment placed farthest into a body lumen from the entrance site.

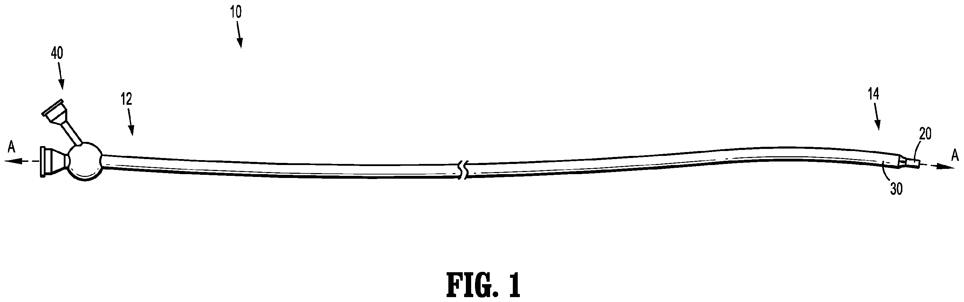

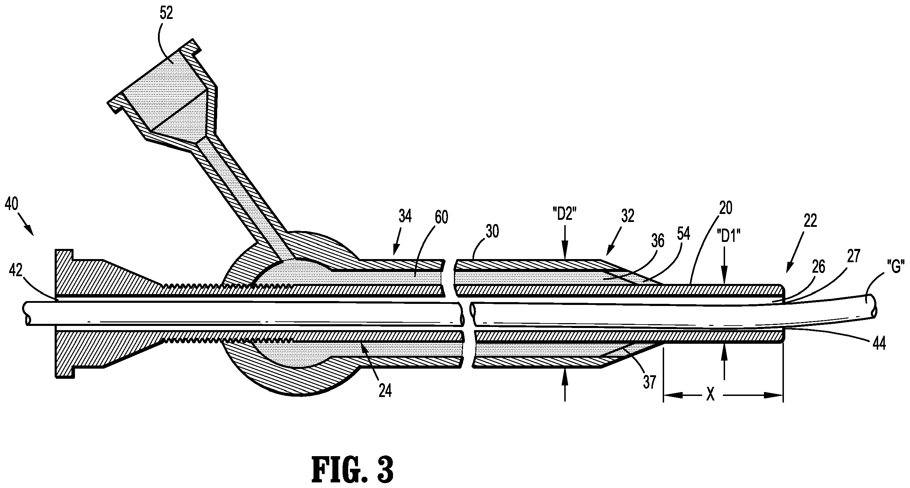

With reference to FIG. 1, a microcatheter 10 can be useful for delivering coils, devices or embolic agents to vascular sites of patients. Though microcatheters may be used to access any neurovascular, peripheral vascular, or cardiovascular treatment site in the body, they are particularly useful for the intravascular treatment of aneurysms or AVMs in the neurovasculature. Microcatheter 10 includes a proximal end 12, a distal end 14, a first flexible tubular body 20 defining longitudinal axis "A-A," and a second flexible tubular body 30. First flexible tubular body 20 includes a distal or leading end 27, a proximal or trailing end 24, and defines a first lumen 26 extending therethrough (see Figure 3). Second flexible tubular body 30 extends substantially parallel to the longitudinal axis "A-A," includes a distal or leading end 37, a proximal or trailing end 34, and defines a second lumen 36 extending therethrough.

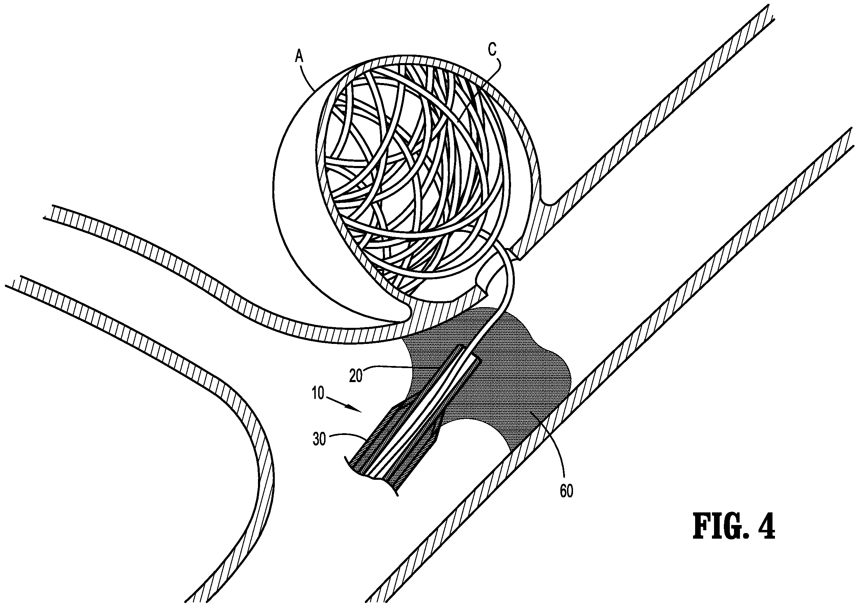

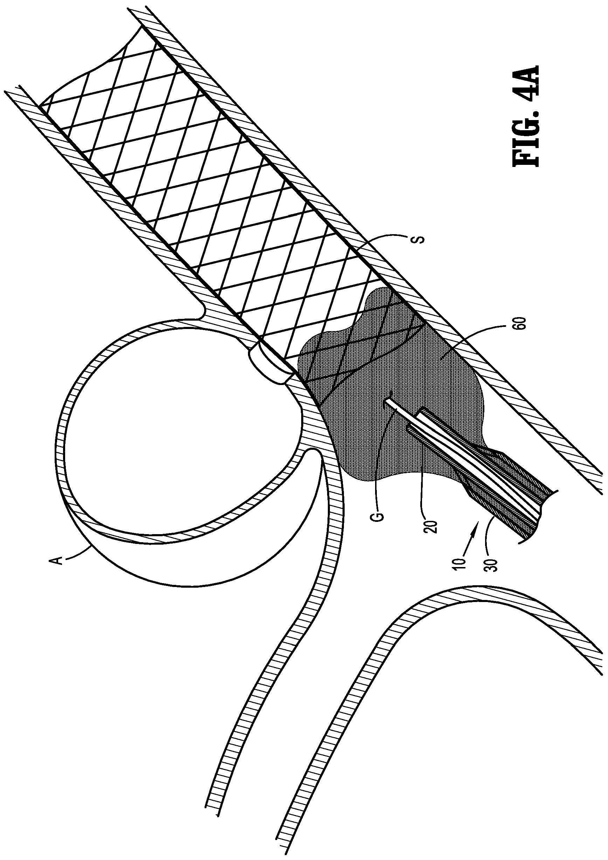

In the accompanying figures, proximal end 12 of microcatheter 10 includes a manifold 40. The illustrated embodiment of manifold 40 includes a first proximal access port 42 in fluid communication with a first distal exit port 44 by way of first lumen 26. First lumen 26 permits the microcatheter 10 to track over a guidewire "G." After removal of the guidewire "G," the first lumen 26 may be used to deliver embolic coils (see FIG. 4) or an embolic agent to the desired vascular site. Manifold 40 also includes a second proximal access portion 52 in fluid communication with a second distal exit port 54 by way of second lumen 36. Second lumen 36 is used to delivery a contrast medium 60 toward a target site within a vessel.

As used herein, the terms "contrast agent" and "contrast medium" refer to both water insoluble and aqueous based contrast agents which are visible by x-ray, fluoroscopy, CT scan, MRI, or the like.

As illustrated, the first flexible tubular body 20 and second flexible tubular body 30 are coaxially disposed. That is, second flexible tubular body 30 is disposed around first flexible tubular body 20 such that longitudinal axis "A-A" extends through a radial center of both tubular bodies 20 and 30. The coaxial orientation of tubular bodies 20 and 30 provides stability of microcatheter 10. In another example, the first flexible tubular body 20 and second flexible tubular body 30 may be arranged in an off axis or eccentric manner

With particular reference to Figure 3, a distal end 27 of first tubular body 20 extends distally beyond a distal end 37 of second lumen 36 by a distance "x." It is envisioned that the distance "x" is between about 2 cm and about 10 cm. In particular, it is envisioned that the distance "x" is between about 5 cm and about 10 cm. It has been determined that the disclosed ranges of distance "x" are sufficient to provide the desired localized delivery of contrast medium while minimizing the amount of contrast media that is necessary for a given procedure, and to allow distal end 27 of first flexible tubular body 20 to be advanced within the vasculature through the reduced profile presented by the smaller diameter of the tubular body 20. Additionally, this disclosed distal offset provides the desired flexibility or "floppiness" of distal end 27 of first tubular body 20 for optimal placement and advancement within the narrow and tortuous paths of the vasculature. As can be appreciated, if first tubular body 20 and second tubular body 30 were conterminous (i.e., distance "x" equal to 0), distal end 27 of first tubular body 20 would not benefit from the disclosed flexibility.

Further, the relative proximity between distal end 27 of first lumen 26 and distal end 37 of second lumen 36 both provides an increased stability of microcatheter 10 along a majority of its length, and also reduces the amount of contrast media required to reach separate catheter lumen is typically used to deliver the contrast media, and the separate catheter lumen is often difficult to advance far enough distally due to the constricted nature of the vasculature. So-called "distal reach" limitations may result in an excess amount of contrast media required to reach the target site, e.g., an aneurysm. Similar "distal reach" limitations may also result when attempting to utilize side-by-side lumens.

It is envisioned that the distance "x" is a fixed distance, such that a physician selects a microcatheter 10 having a desired distance "x" based on a particular procedure and/or the location within the vasculature. It is further envisioned that the distance "x" is a variable distance, such that a user can alter the distance "x," e.g., by sliding second flexible tubular body 30 proximally relative to first flexible tubular body 20 via mechanical structure disposed adjacent manifold 40. Additionally, as shown in Figure 3, first flexible tubular body 20 and second flexible tubular body 30 may be threadably connected, such that a user can rotate second flexible tubular body 30 about first flexible tubular body 20 to change the distance "x." Further, a user can firmly grasp second flexible tubular body 30 and rotate first flexible tubular body 20 about longitudinal axis A-A and with respect to second flexible tubular body 30 to alter the distance "x."

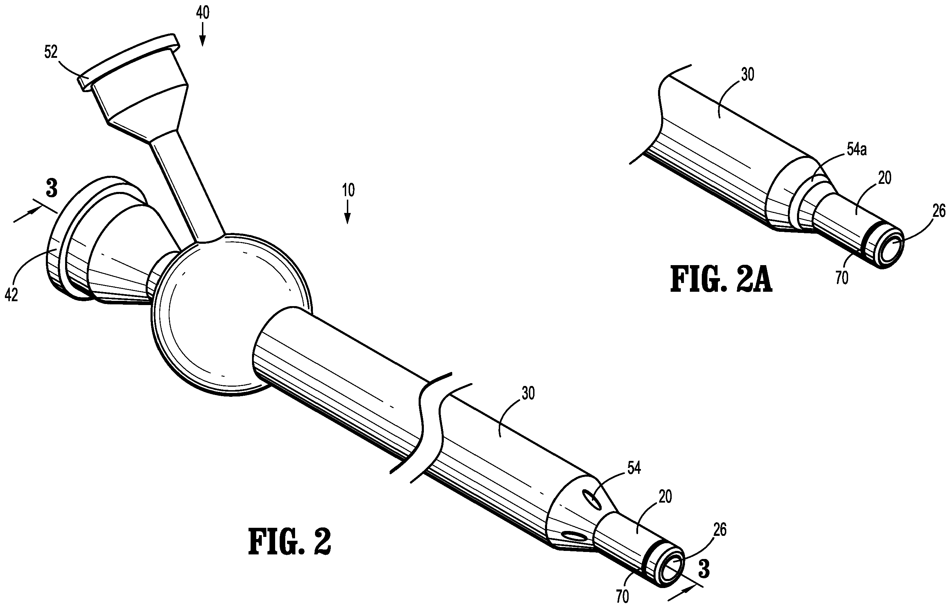

With particular reference to Figure 3, further details of microcatheter 10 are discussed herein. As shown in Figure 3, distal end 37 of second flexible tubular body 30 is tapered. It is envisioned that the taper facilitates atraumatic entry into and traversal through the vasculature. Additionally, other atraumatic and/or low-profile transitions between distal end 37 of second flexible tubular body and first flexible tubular body 20 are envisioned and within the scope of the present disclosure. Additionally, second distal exit port 54 of second lumen 36 may include a plurality of exit ports (Figure 2) or an annular exit port 54a (Figure 2A). The shape of the exits ports may be selected from round, elliptical, or other shapes.

The total length of the microcatheter 10 can generally be in the range of about 150 cm to about 175 cm, although other ranges are also possible. In disclosed embodiments, the outer diameter "Dl" of first flexible tubular body 20 adjacent its distal end 27 is between about 0.020 inches and about 0.030 inches (between about 1F and about 2F), although other ranges are also possible; an outer diameter "D2" of second flexible tubular body 30 adjacent its distal end 37 is between about 0.060 inches and about 0.120 inches (between about 3F and about 6F), although other ranges are also possible. These diameters can be modified appropriately at the proximal and distal ends. Other dimensions than those described herein can be readily utilized by those of ordinary skill in the art in view of the disclosure herein to suit particular intended uses of the microcatheter 10.

The microcatheter 10 may include a marker 70 (Figure 2), for example a radiopaque marker, located adjacent the distal end 14 of the microcatheter 10. The marker 70 can be a ring or band made from a metal or metal alloy, such as platinum, platinum/iridium, gold, nitinol and the like.

Further, it is envisioned that second flexible tubular body 30 is a sheath (i.e., second lumen 36 is defined within a sheath). It is further envisioned that the sheath and first flexible tubular body 20 are made of the same material or different materials, and may include different durometers from each other.

The first and second flexible tubular bodies 20 and 30 can be constructed of a variety of materials and in a variety of ways. It is envisioned that one or both of the first and second flexible tubular bodies 20 and 30 is made from a material selected from the group consisting of Polyurethane, Polyethylene, Polytetrafluoroethylene (PTFE), Expanded Polytetrafluoroethylene (EPTFE), Polyether block amide (including those branded Pebax.RTM), Polyvinyl chloride (PVC), and Polypropylene. In disclosed embodiments, the first and/or second flexible tubular bodies 20 and 30 may be constructed of a material that is compatible with dimethylsulfoxide. The first and second flexible tubular bodies 20 and 30 may also contain zones with varying flexibility which can also be controlled by the methods of construction and materials employed. The first and second flexible tubular bodies 20 and 30 may also be constructed by layering various polymers, such polyimide, polytetrafluoroethylene, polyether block amides, polyamide and the like. The first and second flexible tubular bodies 20 and 30 may additionally include a braid of varying pitches.

It is further envisioned that, when used for the delivery of liquid embolics, the distal end 27 of the first flexible tubular body 20 includes a tip body detachably connected to the first flexible tubular body 20 via a coupling, and which is configured to separate from the first flexible tubular body 20 during use. It is envisioned that the tip body 30 is made from a biocompatible material. What is meant by "biocompatible" is that the material, in the amounts employed, are substantially non-toxic and substantially non-immunogenic when used in the vasculature of a patient. For example, it is envisioned that the tip body is made from a material selected from the group consisting of polyurethane, polyethylene, polytetrafluoroethylene (PTFE), expanded polytetrafluoroethylene (EPTFE), polyether block amide, polyvinyl chloride (PVC), and polypropylene. It is further envisioned that the tip body is made from the same material as the first flexible tubular body 20

In certain embodiments, the tip body can also be "biodegradable." A wide variety of biodegradable/bioerodable and non-biodegradable materials are known which are useful for constructing microcatheter tips. The tip body can be formed of a material which is biodegradable or bioabsorbable in situ. Biodegradable or bioabsorbable materials, or some combination thereof, can be used which allow for the biodegradation/bioabsorption in predetermined conditions.

A variety of biocompatible-biodegradable materials are commercially available and suitable for use in these embodiments. Examples of these materials include DLPLA-poly(dl-lactide), LPLA--poly(l-lactide), PGA--polyglycolide, PDO--poly(dioxanone), PGA-TMC--poly(glycolide-co-trimethylene carbonate), PGA-LPLA-poly(l-lactide-co-glycolide), PGA-DLPLA--poly(dl-lactide-co-glycolide), LPLA-DLPLA--poly(l-lactide-co-dl-lactide), and PDO-PGA-TMC--poly(glycolide-co-trimethylene carbonate-co-dioxanone). Further details of the tip body are disclosed in U.S. Patent Application Serial No. 13/526,611 which was filed on June 19, 2012. It is further envisioned that a lubricious coating may be disposed over components of microcatheter 10, including first and second flexible tubular bodies 20 and 30. Suitable lubricious coatings include hydrophilic materials such as polyvinylpyrrolidone (PVP), polyethylene oxide, polyethylene glycol, cellulosic polymers, and hydrophilic maleic anhydride, or hydrophobic materials such as silicone, PTFE, or FEP. These coatings are typically applied by dip coating or spray methods, and heat or Ultraviolet (UV) curing may be used. For example, cure temperatures up to about 70 degrees C are used for silicone coatings, and several hundred degrees C may be required for PTFE coatings. In addition to the lubricious coating, bioactive coatings may be applied over all or part of the microcatheter 10. Such coatings also may incorporate materials such as heparin, hirudin and its analogs, or other drugs. These coatings typically are applied by dip coating. Bioactive coatings are desirable to prevent blood clotting or for delivery of drugs to a specific site.

With reference to Figures 4 and 4A, the use of the microcatheter 10 within the human body is illustrated. Specifically, the microcatheter 10 is inserted into the patient in a convenient location, such as the groin. A guidewire "G" may be advanced through the first lumen 26 toward the treatment site (e.g., an aneurysm "A"). The microcatheter 10 is advanced through the vasculature (e.g., with the guidewire "G" through the first lumen 26) until the distal end 14 reaches a treatment site, such as for example an AVM or aneurysm "A." The position of the microcatheter 10 can be monitored by visualizing the radiopaque marker 70, for instance. Once the microcatheter 10 is in its appropriate position in the vasculature, an embolic device "C" (e.g., coils or embolic agents) (Figure 4) or a flow diversion device "S" (e.g., stent) (Figure 4A) is delivered to the treatment site through the first lumen 26 (e.g., after removal of the guidewire "G"). The contrast medium 60 is then delivered through the second lumen 36 toward the treatment site to help visualize the relative position of these devices and the progress of the treatment (e.g., if less blood is feeding the aneurysm. As can be appreciated, the contrast medium 60 can also be delivered prior to or during delivery of the devices.

An example of the coils is the Axium™ Detachable Coil System, which is commercially available from Tyco Healthcare Group LP dba Covidien, Irvine, CA.

An example of the embolic agent is Onyx™, a non-adhesive liquid embolic agent comprised of EVOH (ethylene vinyl alcohol) copolymer dissolved in DMSO (dimethyl sulfoxide) and suspended micronized tantalum powder to provide contrast for visualization under fluoroscopy, commercially available from Tyco Healthcare Group LP dba Covidien, Irvine, CA. Further description of suitable embolic agents are described in U.S. Pat. Nos. 5,667,767; 5,695,480; 6,051,607; 6,342,202; 6,531,111; and 6,562,317. An example of the flow diversion device is the PIPELINE™ stent sold by Tyco Healthcare Group LP dba Covidien (Irvine, CA).

After delivery of the embolic device, the microcatheter 10 can be removed from the patient by the application of a retraction force (i.e., a proximally-directed force).

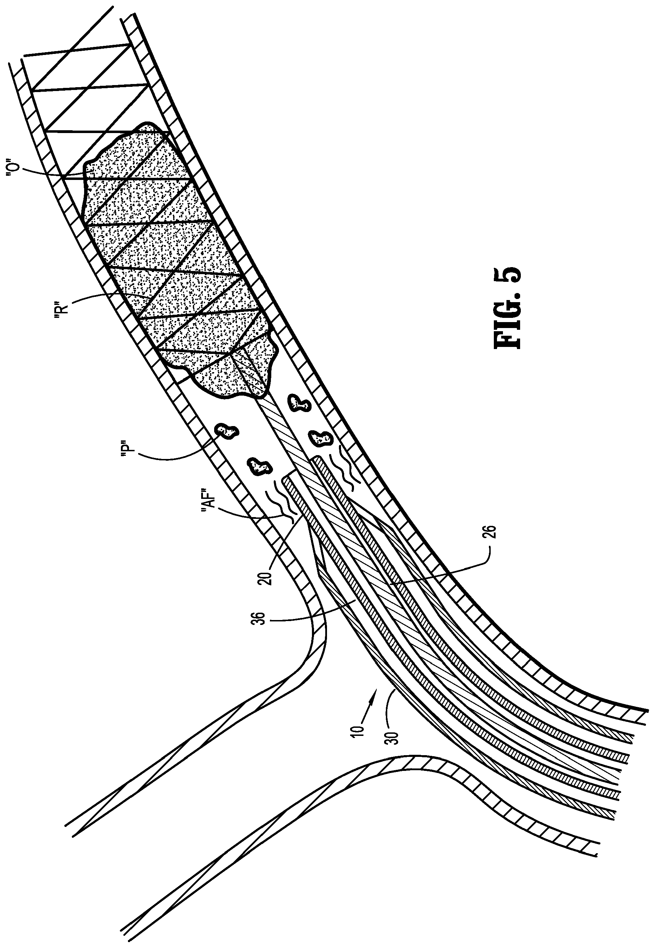

With reference to Figure 5, a schematic view of microcatheter 10 is shown in use adjacent an occlusion "O." Here, microcatheter 10 is used to deploy a stentreiver device "R" from first lumen 26 of microcatheter 10. The stentreiver device "R" is a revascularization device used in part to restore blood flow in the vasculature. In Figure 5, the stentreiver device "R" is being used to break up the occlusion "O" into particulates "P." Second lumen 36 of microcatheter 10 is used for aspiration such that the particulates "P" are carried by the aspiration flow "AF" proximally through second lumen 36.

An example of a stentriever device is the Solitaire FR sold by Tyco Healthcare Group LP dba Covidien (Irvine, CA).

The above description and the drawings are provided for the purpose of describing embodiments of the present disclosure and are not intended to limit the scope of the disclosure in any way. It will be apparent to those skilled in the art that various modifications and variations can be made without departing from the scope of the disclosure. Thus, it is intended that the present disclosure cover the modifications and variations of this disclosure provided they come within the scope of the appended claims and their equivalents.

## Claims

1. A microcatheter (10) comprising:
a first flexible tubular body (20) defining a longitudinal axis (A-A), the body (20) having a proximal end (24), a distal end (27) and a first lumen (26) extending at least partially therethrough; and
a second flexible tubular body (30) extending substantially parallel to the longitudinal axis (A-A) along at least a portion of its length and having a second lumen (36) extending at least partially therethrough, the first lumen (26) and the second lumen (36) being coaxially disposed along at least a majority of the length of the second lumen (36), a distal end of the first lumen (26) extends farther distally than a distal end of the second lumen (36) by a distance x,
wherein the first flexible tubular body and the second flexible tubular body are threadably connected, such that the first and second flexible tubular bodies may be rotated relative to each other to alter the distance x, and **characterised in that** the second flexible tubular body (30) defines a distal exit port (54) in fluid communication with the second lumen (36).

2. The microcatheter (10) of Claim 1, wherein the distance x is between about 2 cm and about 10 cm.

3. The microcatheter (10) of Claim 1, wherein the distance x is between about 5 cm and about 10 cm.

4. The microcatheter (10) of any preceding Claim, further comprising a contrast medium (60) disposed in fluid communication with the second lumen (36), and wherein the second lumen is configured for delivery of the contrast medium (60).

5. The microcatheter (10) of any preceding Claim, wherein the distal end (37) of the second flexible tubular body (30) is tapered.

6. The microcatheter (10) of any preceding Claim, wherein an outer diameter of the second flexible tubular body (30) is between about 1.524mm (0.060 inches) and about 2.032mm (0.080 inches).

7. The microcatheter (10) of any preceding Claim, further comprising a tip body detachably connected to the first tubular body (20) via a coupling.

8. The microcatheter (10) of any preceding Claim, wherein the distal end (37) of the second flexible tubular body (30) includes a plurality of exit ports (34).

9. The microcatheter (10) of any preceding Claim, wherein the distal end (37) of the second flexible tubular body (30) includes an annular exit port (54a).

10. The microcatheter (10) of any preceding Claim, wherein the second flexible tubular body (30) is defined within a sheath.

11. The microcatheter (10) of Claim 10, wherein the second flexible tubular body (30) and the sheath are made of the same material.

12. The microcatheter (10) of Claim 11, wherein the second flexible tubular body (30) and the sheath have different durometers.

## Patentansprüche

1. Mikrokatheter (10), umfassend:
einen ersten flexiblen Hohlkörper (20), der eine Längsachse (A-A) festlegt, wobei der Hohlkörper (20) ein proximales Ende (24), ein distales Ende (27) und ein erstes Lumen (26) aufweist, das sich wenigstens teilweise dadurch erstreckt; und
einen zweiten flexiblen Hohlkörper (30), der sich im Wesentlichen parallel zu der Längsachse (A-A) entlang wenigstens eines Teils seiner Länge erstreckt und ein zweites Lumen (36) aufweist, das sich wenigstens teilweise dadurch erstreckt, wobei das erste Lumen (26) und das zweite Lumen (36) entlang wenigstens eines Großteils der Länge des zweiten Lumens (36) koaxial angeordnet sind, und ein distales Ende des ersten Lumens (26) sich um einen Abstand x weiter distal als das distale Ende des zweiten Lumens (36) erstreckt,
wobei der erste flexible Hohlkörper und der zweite flexible Hohlkörper einfädelbar verbunden sind, sodass der erste und der zweite flexible Hohlkörper relativ zueinander gedreht werden können, um den Abstand x zu verändern, und **dadurch gekennzeichnet, dass** der zweite flexible Hohlkörper (30) ein distales Austrittsfenster (54) in Fluidkommunikation mit dem zweiten Lumen (36) festlegt.

2. Mikrokatheter (10) nach Anspruch 1, wobei der Abstand x zwischen etwa 2 cm und etwa 10 cm beträgt.

3. Mikrokatheter (10) nach Anspruch 1, wobei der Abstand x zwischen etwa 5 cm und etwa 10 cm beträgt.

4. Mikrokatheter (10) nach einem der vorhergehenden Ansprüche, der weiter ein Kontrastmittel (60) umfasst, das in Fluidkommunikation mit dem zweiten Lumen (36) angeordnet ist, und wobei das zweite Lumen zur Abgabe des Kontrastmediums (60) ausgelegt ist.

5. Mikrokatheter (10) nach einem der vorhergehenden Ansprüche, wobei das distale Ende (37) des zweiten flexiblen Hohlkörpers (30) sich verjüngt.

6. Mikrokatheter (10) nach einem der vorhergehenden Ansprüche, wobei ein Außendurchmesser des zweiten flexiblen Hohlkörpers (30) zwischen etwa 1,524 mm (0,060 Zoll) und etwa 2,032 mm (0,080 Zoll) liegt.

7. Mikrokatheter (10) nach einem der vorhergehenden Ansprüche, der weiter einen Spitzenkörper umfasst, der abnehmbar über eine Ankopplung mit dem ersten Hohlkörper (20) verbunden ist.

8. Mikrokatheter (10) nach einem der vorhergehenden Ansprüche, wobei das distale Ende (37) des zweiten flexiblen Hohlkörpers (30) eine Vielzahl von Austrittsfenstern (34) umfasst.

9. Mikrokatheter (10) nach einem der vorhergehenden Ansprüche, wobei distale Ende (37) des zweiten flexiblen Hohlkörpers (30) ein ringförmiges Austrittsfenster (54a) umfasst.

10. Mikrokatheter (10) nach einem der vorhergehenden Ansprüche, wobei der zweite flexible Hohlkörper (30) innerhalb eines Schafts festgelegt ist.

11. Mikrokatheter (10) nach Anspruch 10, wobei der zweite flexible Hohlkörper (30) und der Schaft aus demselben Material bestehen.

12. Mikrokatheter (10) nach Anspruch 11, wobei der zweite flexible Hohlkörper (30) und der Schaft unterschiedliche Durometer aufweisen.

## Revendications

1. Microcathéter (10) comprenant :
un premier corps tubulaire flexible (20) définissant un axe longitudinal (A-A), le corps (20) ayant une extrémité proximale (24), une extrémité distale (27) et une première lumière (26) s'étendant au moins partiellement à travers celui-ci ; et
un second corps tubulaire flexible (30) s'étendant sensiblement parallèlement à l'axe longitudinal (A-A) le long d'au moins une partie de sa longueur et ayant une seconde lumière (36) s'étendant au moins partiellement à travers celui-ci, la première lumière (26) et la seconde lumière (36) étant coaxialement disposées le long d'au moins une majorité de la longueur de la seconde lumière (36), une extrémité distale de la première lumière (26) s'étend distalement plus loin qu'une extrémité distale de la seconde lumière (36) d'une distance x,
dans lequel le premier corps tubulaire flexible et le second corps tubulaire flexible sont raccordés par filetage, de telle sorte que les premier et second corps tubulaires flexibles peuvent être mis en rotation l'un par rapport à l'autre pour modifier la distance x, et **caractérisé en ce que** le second corps tubulaire flexible (30) définit un orifice de sortie distal (54) en communication fluidique avec la seconde lumière (36).

2. Microcathéter (10) selon la revendication 1, dans lequel la distance x est comprise entre environ 2 cm et environ 10 cm.

3. Microcathéter (10) selon la revendication 1, dans lequel la distance x est comprise entre environ 5 cm et environ 10 cm.

4. Microcathéter (10) selon l'une quelconque des revendications précédentes, comprenant en outre un milieu de contraste (60) disposé en communication fluidique avec la seconde lumière (36), et dans lequel la seconde lumière est configurée pour l'administration du milieu de contraste (60).

5. Microcathéter (10) selon l'une quelconque des revendications précédentes, dans lequel l'extrémité distale (37) du second corps tubulaire flexible (30) est conique.

6. Microcathéter (10) selon l'une quelconque des revendications précédentes, dans lequel un diamètre extérieur du second corps tubulaire flexible (30) est compris entre environ 1,524 mm (0,060 pouce) et environ 2,032 mm (0,080 pouce).

7. Microcathéter (10) selon l'une quelconque des revendications précédentes, comprenant en outre un corps de pointe raccordé de façon détachable au premier corps tubulaire (20) via un couplage.

8. Microcathéter (10) selon l'une quelconque des revendications précédentes, dans lequel l'extrémité distale (37) du second corps tubulaire flexible (30) comprend une pluralité d'orifices de sortie (34).

9. Microcathéter (10) selon l'une quelconque des revendications précédentes, dans lequel l'extrémité distale (37) du second corps tubulaire flexible (30) comprend un orifice de sortie annulaire (54a).

10. Microcathéter (10) selon l'une quelconque des revendications précédentes, dans lequel le second corps tubulaire flexible (30) est défini à l'intérieur d'une gaine.

11. Microcathéter (10) selon la revendication 10, dans lequel le second corps tubulaire flexible (30) et la gaine sont fabriqués avec le même matériau.

12. Microcathéter (10) selon la revendication 11, dans lequel le second corps tubulaire flexible (30) et la gaine ont des duromètres différents.
